# EUROPEAN PATENT APPLICATION

(11) **EP 1 964 851 A1**
(43) Date of publication of application: **03.09.2008**
(21) Application number: 08005286.3
(22) Date of filing: 13.11.2002
(51) Int. Cl.: C07K 16/12, A61K 39/40, A61P 31/04, A61K 38/14

(54) **Treatment of micro-organism infection**

(30) Priority: 22.11.2001 GB 0127983
(62) Divisional of application: 02777534.5
(71) Applicant: Neutec Pharma Limited, Camberley, Surrey SG16 7SR (GB)
(72) Inventor: Burnie, James Peter, Cheshire SK9 7PY (GB); Matthews, Ruth Christine, Alderley Edge SK9 7PY Cheshire (GB)
(74) Representative: Arends, William Gerrit

(57) **Abstract**

A medicament comprising a therapeutically effective quantity of daptomycin and an antibody. The antibody is specific against the epitope displayed by the peptide having the amino acid sequence of SEQ. ID NO: 3.

SEQUENCE LISTING

<110>  NeuTec Pharma Ltd

<120>  Treatment of Micro-Organism Infection

<130>  MEPP020308A

<150>  GB0127983.5
<151>  2001-11-22

<150>  EP02777534.5
<151>  2002-11-13

<160>  3

<170>  PatentIn version 3.3

<210>  1
<211>  795
<212>  DNA
<213>  Artificial

<220>
<223>  Human single chain (scFv) recombinant antibody


<220>
<221>  CDS
<222>  (1)..(792)

<400>  1
atg gcc aag gtg cag ctg ttg cag tct gca act gag gtg aag aag cct      48
Met Ala Lys Val Gln Leu Leu Gln Ser Ala Thr Glu Val Lys Lys Pro
1               5                   10                  15

ggg gcc tca gtg aag gtc tcc tgc aag gct tat ggt tac act ttc acc      96
Gly Ala Ser Val Lys Val Ser Cys Lys Ala Tyr Gly Tyr Thr Phe Thr
            20                  25                  30

gat tat ggt ata acc tgg gtg cga cag gcc cct gga caa ggg ctt gag      144
Asp Tyr Gly Ile Thr Trp Val Arg Gln Ala Pro Gly Gln Gly Leu Glu
        35                  40                  45

tgg atg gga tgg atc agc gct tat aat ggt tac aca aac tat gcg cag      192
Trp Met Gly Trp Ile Ser Ala Tyr Asn Gly Tyr Thr Asn Tyr Ala Gln
    50                  55                  60

aag ttc cag gac aga atc acc atg acc aca gac gca tcc acg agc aca      240
Lys Phe Gln Asp Arg Ile Thr Met Thr Thr Asp Ala Ser Thr Ser Thr
65                  70                  75                  80

gcc tat atg gag ttg agg agc ctg aga cct gac gac acg gcc gta ctt      288
Ala Tyr Met Glu Leu Arg Ser Leu Arg Pro Asp Asp Thr Ala Val Leu
                85                  90                  95

tac tgt gcg agg gat cgg gaa gat gca tct ctg tta cgg gac ggt cac      336
Tyr Cys Ala Arg Asp Arg Glu Asp Ala Ser Leu Leu Arg Asp Gly His

            100                 105                 110

tgg ggc cag ggc acc ctg gtc acc gtg agc tct ggt gga ggc ggt tca 384
Trp Gly Gln Gly Thr Leu Val Thr Val Ser Ser Gly Gly Gly Gly Ser
        115                 120                 125

ggc gga ggc ggt tca ggc gga ggt ggc agc ggc ggt ggc gga tcg gaa 432
Gly Gly Gly Gly Ser Gly Gly Gly Gly Ser Gly Gly Gly Gly Ser Glu
    130                 135                 140

acg aca ctc acg cag tct cca ggc acc ctg tct ttg tct cca ggg gaa 480
Thr Thr Leu Thr Gln Ser Pro Gly Thr Leu Ser Leu Ser Pro Gly Glu
145                 150                 155                 160

agg gcc acc ctc tcc tgc agg gcc agt cag att gtt agc agc agc tac 528
Arg Ala Thr Leu Ser Cys Arg Ala Ser Gln Ile Val Ser Ser Ser Tyr
                165                 170                 175

tta gcc tgg tac cag cag aaa cct ggc cag gct ccc agg ctc ctc atc 576
Leu Ala Trp Tyr Gln Gln Lys Pro Gly Gln Ala Pro Arg Leu Leu Ile
            180                 185                 190

tat ggt gca tcc agc agg gcc act ggc atc cca gac agg ttc agt ggc 624
Tyr Gly Ala Ser Ser Arg Ala Thr Gly Ile Pro Asp Arg Phe Ser Gly
        195                 200                 205

agt ggg tct ggg aca gac ttc act ctc acc atc agc aaa ctg gag cct 672
Ser Gly Ser Gly Thr Asp Phe Thr Leu Thr Ile Ser Lys Leu Glu Pro
    210                 215                 220

gaa gat ttt gca gtg tat tac tgt cag cat tat ggt agc tca tct ccg 720
Glu Asp Phe Ala Val Tyr Tyr Cys Gln His Tyr Gly Ser Ser Ser Pro
225                 230                 235                 240

tgg acg tct cgg cca agg gac caa agt gat atc aaa cgt gcg gcc gca 768
Trp Thr Ser Arg Pro Arg Asp Gln Ser Asp Ile Lys Arg Ala Ala Ala
                245                 250                 255

ctc gag cac cac cac cac cac cac tga 795
Leu Glu His His His His His His
            260

<210> 2
<211> 264
<212> PRT
<213> Artificial

<220>
<223> Synthetic Construct

<400> 2

Met Ala Lys Val Gln Leu Leu Gln Ser Ala Thr Glu Val Lys Lys Pro
1               5                   10                  15

Gly Ala Ser Val Lys Val Ser Cys Lys Ala Tyr Gly Tyr Thr Phe Thr

20                  25                  30

Asp Tyr Gly Ile Thr Trp Val Arg Gln Ala Pro Gly Gln Gly Leu Glu
        35                  40                  45

Trp Met Gly Trp Ile Ser Ala Tyr Asn Gly Tyr Thr Asn Tyr Ala Gln
    50                  55                  60

Lys Phe Gln Asp Arg Ile Thr Met Thr Thr Asp Ala Ser Thr Ser Thr
65                  70                  75                  80

Ala Tyr Met Glu Leu Arg Ser Leu Arg Pro Asp Asp Thr Ala Val Leu
                85                  90                  95

Tyr Cys Ala Arg Asp Arg Glu Asp Ala Ser Leu Leu Arg Asp Gly His
            100                 105                 110

Trp Gly Gln Gly Thr Leu Val Thr Val Ser Ser Gly Gly Gly Gly Ser
        115                 120                 125

Gly Gly Gly Gly Ser Gly Gly Gly Gly Ser Gly Gly Gly Gly Ser Glu
    130                 135                 140

Thr Thr Leu Thr Gln Ser Pro Gly Thr Leu Ser Leu Ser Pro Gly Glu
145                 150                 155                 160

Arg Ala Thr Leu Ser Cys Arg Ala Ser Gln Ile Val Ser Ser Ser Tyr
                165                 170                 175

Leu Ala Trp Tyr Gln Gln Lys Pro Gly Gln Ala Pro Arg Leu Leu Ile
            180                 185                 190

Tyr Gly Ala Ser Ser Arg Ala Thr Gly Ile Pro Asp Arg Phe Ser Gly
        195                 200                 205

Ser Gly Ser Gly Thr Asp Phe Thr Leu Thr Ile Ser Lys Leu Glu Pro
    210                 215                 220

Glu Asp Phe Ala Val Tyr Tyr Cys Gln His Tyr Gly Ser Ser Ser Pro
225                 230                 235                 240

Trp Thr Ser Arg Pro Arg Asp Gln Ser Asp Ile Lys Arg Ala Ala Ala
                245                 250                 255

Leu Glu His His His His His His
            260

<210>  3
<211>  7
<212>  PRT
<213>  Staphylococcus aureus

<400>  3

Gly Val Thr Thr Ser Leu Ser
1               5

## Description

The present invention is concerned with the treatment of infections of the human or animal body by micro-organisms such as *S. aureus,* particularly antibiotic resistant strains of the organisms such as MRSA. Provided are medicaments for the treatment of infection by micro-organisms such as *S*. *aureus,* methods of manufacture of medicaments for the treatment of infection, pharmaceutical packs containing medicaments for the treatment of infection, and methods of treatment of the human or animal body for infection by said micro-organisms.

Multiple drug resistance (MDR) is an increasing problem amongst gram-positive bacteria (Banergee, S.N. et al. 1991, Am. J. Med. 91: 865-895; Shaberg, D.R. et al., 1991, Am. J. Med. suppl., 88: 72-75; Gaynes, R.P, et al., 1994, Infect. Dis. Clin. Pract., 6: 452-455), particularly in hospitals. In particular, methicillin-resistant *Staphylococcus aureus* (MRSA) and coagulase-negative staphylococci (CNS), particularly methicillin-resistant CNS, prove problematic, being resistant to all penicillins and cephalosporins. Resistance to other agents such as quinolones is widespread (Malabarta, A. et al., 1997, Eur. J. Med. Chem., 32: 459-478; Lewis, K., 1994, TIBS, 19: 119-123; Traub, W.H. et al., 1996, Chemotherapy, 42: 118-132). Treatment is typically effected using vancomycin or teicoplanin. However, resistance to these agents is spreading and so new therapies are needed. For example, Hubert SK et al., (J Clin Microbiol. 1999 Nov;37(11):3590-3; PMID: 10523558) discusses *S. aureus* isolates with reduced susceptibility to vancomycin and teicoplanin. It has also been fund that certain epidemic strains of MRSA, including EMRSA-15 (www.phls.org.uk, Public Health Laboratories, Colindale, UK), can give rise to subclones with increased resistance to vancomycin (Burnie J et al., Clin Infect Dis. 2000 Sep;31(3):684-9; PMID: 11017816). It was hoped that linezolid would provide a much needed alternative to current therapies, but Tsiodras S. et al. (Lancet. 2001 Jul 21;358(9277):207-8; PMID: 11476839) have reported a clinical isolate of MRSA resistant to linezolid in a patient being treated with this antibiotic for dialysis associated peritonitis.

In addition administration of large quantities of such antibiotics to patients to effect treatment of infections, especially of infections which display resistance to the antibiotic(s) being administered, can be cytotoxic and nephrotoxic and although helping to save the patient's life can result in severe side-effects.

The treatment and diagnosis of Staphylococcal infections is discussed widely in the prior art and particularly relevant publications include WO 98/01154, WO 99/50418, and EP 0786519. The causes of antibiotic resistance are also discussed in publications such as Allignet J. (Gene 1997 Nov 20; 202(1-2):133-8; PMID: 9427556).

Thus there is a clear need for new and enhanced therapies for micro-organism, particularly *S aureus,* infection.

The present inventors have now found that a particular combination of agents, namely an antibody (detailed below) having a novel antigen binding part and a glycopeptide antibiotic (such as vancomycin or teicoplanin), has a very surprising synergistic therapeutic effect - the therapeutic efficacy of the glycopeptide antibiotic is substantially enhanced when compared to its therapeutic efficacy when used alone. This is particularly the case when the micro-organism being treated is resistant to the glycopeptide antibiotic. **Importantly, the present invention is able to effect treatment of fully vancomycin resistant and vancomycin intermediately resistant strains of *S*. *aureus* using vancomycin together with the antibody of SEQ ID NO: 2.** Nowhere in the prior art is it suggested that this might be achieved. The glycopeptide antibiotics work by affecting the bacterial cell wall. Other antibiotics such as the penicillins also affect the bacterial cell wall. However, the present inventors have found that their efficacy is not improved by using them with the antibody of SEQ ID NO: 2. in particular, experiments have shown that the efficacy of flucloxacillin is not improved by using it with the antibody of SEQ ID NO: 2. Bacteria resistant to flucloxacillin did not have their resistance reduced by using the antibody of SEQ ID NO: 2.

According to the present invention there is provided an antibody having the sequence of SEQ ID NO: 2 or an antigen binding fragment thereof. The antibody is encoded by the nucleotide sequence of SEQ ID NO: 1 although of course the nucleotide sequence can be readily modified to e.g. optimise the codons for specific micro-organisms used to synthesise it or for other reasons as desired.

The antibody of SEQ ID NO: 2 (also referred to as "Aurograb" RTM) is a human genetic recombinant antibody. It binds to the immunodominant cell surface antigen, GrfA, a staphylococcal ABC transporter protein (WO 99/50418; Burnie JP et al., Infect Immun. 2000 Jun;68(6):3200-9; PMID: 10816464). It was originally derived from patients who had recovered from *S. aureus* septicaemia and were producing antibodies to GrfA. It has intrinsic anti-staphylococcal activity and shows synergy with vancomycin and other glycopeptide antibiotics, both *in vitro* and *in vivo,* against a wide range of *S. aureus* strains including MRSA and vancomycin-resistant MRSA. The antibacterial activity of the antibody of SEQ ID NO: 2, both alone and in combination with glycopeptide antibiotics, is of significant benefit in the treatment of *S. aureus* infections.

Both human plasma-derived immunoglobulin (Ramisse F et al., J Infect Dis. 1993 Oct;168(4):1030-3; PMID: 8376815) and rabbit hyperimmune antiserum raised against GrfA are protective in models of staphylococcal infections (see "Experiments" section below). Human recombinant antibodies specific for GrfA are protective in mouse models of MRSA infection. A particular amino acid sequence of GrfA, the epitope of SEQ ID NO: 3, was found to be frequently targeted by the humoral immune response in patients recovering from *S. aureus* septicaemia (Burnie JP *et al.,* 2000 *supra*).

However, nowhere in the prior art is it suggested that the improved treatment of micro-organism infections such as *S. aureus* may be effected using a combination of a glycopeptide antibiotic such as vancomycin, teicoplanin and daptomycin, and an anti-GrfA antibody, particularly not antibody specific to the epitope of SEQ ID NO: 3, and more particularly not the antibody having the sequence of SEQ ID NO: 2.

Thus according to the present invention there is provided a medicament comprising a therapeutically effective quantity of a glycopeptide antibiotic and antibody specific against GrfA, particularly antibody specific against the epitope of SEQ ID NO: 3. Also provided are methods of manufacture of medicaments, pharmaceutical packs and methods of treatment all comprising or using same. The medicament may be for the treatment of *S. aureus* infection. The term "treatment" as used herein means any treatment which is designed to cure, alleviate, remove or lessen the symptoms of, or prevent or reduce the possibility of contracting such an infection, and includes prophylaxis.

The GrfA protein, which is an ABC transporter protein, has been isolated and purified from *S. aureus,* but homologues which perform the same function in other organisms exist and they may be used as the target for therapy. Thus antibody can be prepared against a homolog of GrfA produced by a micro-organism other than *S. aureus* and may be used together with a glycopeptide antibiotic to effect treatment of infection by the micro-organism in a patient. Examples of other micro-organisms in which treatment can be effected are other Staphylococci, particularly coagulase negative Staphylococci, such as *S. haemolyticus, S. epidermidis* and *S*. *saprophyticus.* Infection by Enterococci, particularly *E. faecalis* and *E*. *faecium*, can also be treated, as can infection by Corynebacteria such as *C. jeikeium* and *C. xerosis*.

The homolog of GrfA may have a sequence homology of at least 60%, for example at least 70, 80, 85, 90, 95, 96, 97, 98, 99 or 99.5% with GrfA. Sequence homologies are determined using the NCBI BLASTN (nucleotide sequence comparisons) or BLASTP (polypeptide comparisons) programs, Version 2.1.2, with default parameters. The NCBI BLAST programs is to be found at www.ncbi.nlm.nih.gov/blast/. The term sequence identity used herein refers to amino acid residues in optimally aligned sequences which match exactly at corresponding relative positions.

In organisms other than *S*. *aureus*, particularly other Staphylococci, the epitope (SEQ ID NO: 3) against which antibodies are raised and which are used as the basis for the treatment may remain the same.

Thus according to the present invention there is provided a medicament for the treatment of infection, particularly *S. aureus* infection, said medicament comprising a therapeutically effective quantity of a glycopeptide antibiotic and the antibody of SEQ ID NO: 2 or an antigen binding fragment thereof.

Also provided is a glycopeptide antibiotic and the antibody of SEQ ID NO: 2 or an antigen binding fragment thereof for use in a method of manufacture of a medicament for the treatment of infection.

In effecting a therapy against infection, particularly *S. aureus* infection, the glycopeptide antibiotic and antibody or antigen binding fragment may be administered to a patient separately or sequentially. Also provided according to the present invention is a pharmaceutical pack comprising a therapeutically effective quantity of a glycopeptide antibiotic and the antibody of SEQ ID NO: 2 or an antigen binding fragment thereof.

Also provided according to the present invention is the use of a glycopeptide antibiotic and the antibody of SEQ ID NO: 2 or an antigen binding fragment thereof for the manufacture of a medicament for the treatment of infection, particularly *S. aureus* infection.

Also provided according to the present invention is a method of treating infection, particularly *S. aureus* infection, in a patient, comprising the step of administering to said patient a therapeutically effective quantity of a glycopeptide antibiotic and the antibody of SEQ ID NO: 2 or an antigen binding fragment thereof.

Glycopeptide antibiotics of particular usefulness in the present invention are vancomycin, teicoplanin and daptomycin, although of course the present invention extends to other members of the family of glycopeptide antibiotics.

The term "antibody" in its various grammatical forms is used herein to refer to immunoglobulin molecules and immunologically active portions of immunoglobulin molecules, i.e., molecules that contain an antibody combining site or paratope. Such molecules are also referred to as "antigen binding fragments" of immunoglobulin molecules.

Illustrative antibody molecules are intact immunoglobulin molecules, substantially intact immunoglobulin molecules and those portions of an immunoglobulin molecule that contain the paratope, including those portions known in the art as Fab, Fab', F(ab')2, scFv and F(v).

The term "antibody combining site" refers to that structural portion of an antibody molecule comprised of a heavy and light chain variable and hypervariable regions that specifically binds (immunoreacts with) antigen.

As regards the antibody of SEQ ID NO: 2, it may be readily modified to alter its amino acid sequence whilst presenting the same paratope and retaining its antigen binding specificity. Generally speaking, its structure is arranged (amino to carboxy terminal sequence) as a human immunoglobulin variable heavy domain (V_{H}) and variable light chain (V_{K}) covalently joined together by a linker and having a His carboxy-terminal sequence.

Each of the variable regions is comprised of the complement determining regions CDR₁, CDR₂ and CDR₃, and they are fundamental in defining the antigen binding specificity of the antibody, i.e. the paratope. The present inventors have found that of these regions, the CDR₃ part of the V_{H} region is the most important in defining antigen specificity. Further teachings about antibodies are widely available in the art, e.g. Harlow, E. and Lane, D., "Using Antibodies: A Laboratory Manual", Cold Spring Harbor Laboratory Press, New York, 1998. With the common general knowledge about the paratope defining portions of an antibody combining site and the above teachings, a person skilled in the art is readily able to modify the sequence of SEQ ID NO: 2 to produce alternative antibodies with the same paratope and which enable one to achieve the same or similar therapeutic effect.

For example an antigen binding fragment of the antibody of SEQ ID NO: 2 may be readily prepared by simply removing one or more of the carboxy-terminal His residues. Other examples of modifications include the grafting of the hypervariable (complement determining regions) of the antibody of SEQ ID NO: 2 into variable framework regions different to those of SEQ ID NO: 2 such that the resultant antibody still has the same paratope (see e.g. EP 239400 and suchlike).

By "improved treatment" is meant that a given quantity of a glycopeptide antibiotic has a greater therapeutic effect when administered to a patient together with the antibody of the present invention than when administered alone. Similarly, a desired therapeutic effect can be achieved in a patient with a smaller dosage of a glycopeptide antibiotic when administered to a patient together with the antibody of the present invention than when the glycopeptide antibiotic is administered alone. This is particularly the case when the micro-organism such as *S. aureus* is resistant to the glycopeptide antibiotic. This can be extremely useful in reducing any side effects of treatment.

By providing existing glycopeptide antibiotics with a "second wind" and allowing them to be therapeutically effective against e.g. *S. aureus* infection, even when the *S. aureus* is resistant to them, the present invention provides medicaments and treatments whose safety and efficacy can be readily evaluated and which do not involve new classes of substances - the safety parameters for glycopeptide antibiotics are already well known and can be applied to the present invention. Similarly the use of antibodies as the other active ingredient in the present invention is a relatively simple safety issue. In particular, when the antibody is in the form of a recombinant antibody fragment derived from human antibody sequences, its immunogenicity will be extremely low. In addition the use of treatment regimes in which the antibody is administered over a very short time period (for example a few days) means that there is little opportunity for the patient's immune system to develop an immune response against it. Thus adverse hypersensitivity-type reactions caused by the production of antibody against the antibody of the present invention and the formation/deposition of immune complexes is very small.

The antibody of SEQ ID NO: 2 also has a number of specific advantages - it does not have the F_{C} portion which can trigger complement deposition and macrophage binding and hence is unlikely to cause inappropriate activation of the complement cascade which can cause inflammation and tissue damage.

By producing the antibodies of the present invention without the use of animal-derived products, the possibility of introducing human or animal infectious disease agents or oncogenes into patients can be avoided.

Treatment regimes and dosages of the medicaments of the present invention are described below, and modification of them will be readily apparent to a person skilled in the art, who in particular will be aware of treatment regimes used with the glycopeptide antibiotic and will be able to modify them appropriately. For example, simple dose-response assays can be readily used and results of treatment in mammalian models such as murine models can be simply extrapolated across to humans.

The present invention will be further apparent from the following description with reference to the accompanying Figure, which shows by way of example only forms of treatment of S. aureus infection.

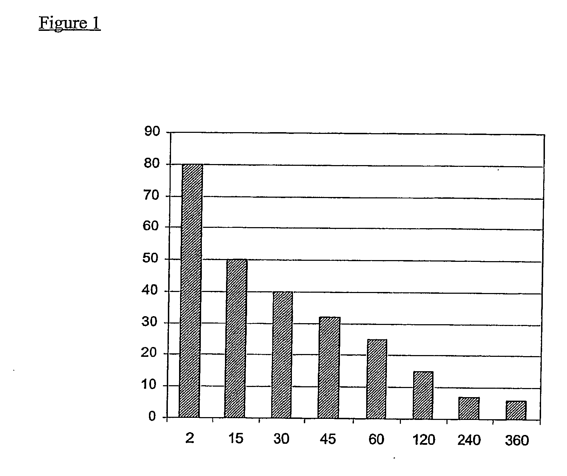

- Figure 1: shows the pharmacokinetics of high doses of Aurograb. X-axis is time in minutes. Y-axis is µg/ml of Aurograb in blood samples.

### EXPERIMENTS

The experiments detailed below show that Aurograb (i.e. SEQ ID NOs: 1 and 2), the human recombinant antibody fragment of the present invention specific against GrfA, has intrinsic anti-staphylococcal activity *in vitro* and in murine staphylococcal infection and that it shows synergistic activity with glycopeptide antibiotics, particularly the broad spectrum glycopeptide antibiotic vancomycin.

The synergistic activity with glycopeptide antibiotics is manifested by:
(1) a reduction in the minimum inhibitory concentration of vancomycin in the presence of Aurograb *in vitro* and
(2) reduced organ colony counts in murine models of *S. aureus* infection, when, after challenge with *S. aureus,* a single dose of Aurograb (2 mg/kg) is given in conjunction with a single dose of vancomycin (6 mg/kg).

Unless stated otherwise, all procedures detailed herein were performed using standard protocols and following manufacturer's instructions where applicable. Standard protocols for various techniques including PCR, molecular cloning, manipulation and sequencing, the manufacture of antibodies, epitope mapping and mimotope design, cell culturing and phage display, are described in texts such as McPherson, M.J. et al. (1991, PCR: A practical approach, Oxford University Press, Oxford), Sambrook, J. et al. (1989, Molecular cloning: a laboratory manual, Cold Spring Harbour Laboratory, New York), Huynh and Davies (1985, "DNA Cloning Vol I - A Practical Approach", IRL Press, Oxford, Ed. D.M. Glover), Sanger, F. et al. (1977, PNAS USA 74(12): 5463-5467), Harlow, E. and Lane, D. ("Using Antibodies: A Laboratory Manual", Cold Spring Harbor Laboratory Press, New York, 1998), Jung, G. and Beck-Sickinger, A.G. (1992, Angew. Chem. Int. Ed. Eng., 31: 367-486), Harris, M.A. and Rae, I.F. ("General Techniques of Cell Culture", 1997, Cambridge University Press, ISBN 0521 573645), "Phage Display of Peptides and Proteins: A Laboratory Manual" (Eds. Kay, B.K., Winter, J., and McCafferty, J., Academic Press Inc., 1996, ISBN 0-12-402380-0).

Reagents and equipment useful in, amongst others, the methods detailed herein are available from the likes of Amersham (www.amersham.co.uk), Boehringer Mannheim (www.boehringer-ingeltheim.com), Clontech (www.clontech.com), Genosys (www.genosys.com), Millipore (www.millipore.com), Novagen (www.novagen.com), Perkin Elmer (www.perkinelmer.com), Pharmacia (www.pharmacia.com), Promega (www.promega.com), Qiagen (www.qiagen.com), Sigma (www.sigma-aldrich.com) and Stratagene (www.stratagene.com).

Where "PMID" reference numbers are given for publications, these are the PubMed identification numbers allocated to them by the US National Library of Medicine, from which full bibliographic information and abstract for each publication is available at www.ncbi.nlm.nih.gov. This can also provide direct access to electronic copies of the complete publications, particularly in the case of e.g. PNAS, JBC and MBC publications.

The contents of each of the references discussed herein, including the references cited therein, are herein incorporated by reference in their entirety.

### 1. Human Study

For this study, Aurograb is given in conjunction with vancomycin to reduce the mortality and/or morbidity of patients with culture-confirmed deep-seated MRSA infection. This study is performed in three parts:
i) A tolerance and dose ranging study in which cohorts of 3 patients with MRSA infection are given staged escalating doses of Aurograb, starting with a sub-therapeutic dose (0.1 mg/kg) and rising to 1 mg/kg b.d., during which each patient is carefully monitored for any adverse side effects.
ii) An interim, open, labelled study in which 5 patients with MRSA infection are given the anticipated therapeutic course of Aurograb, namely 1 mg/kg b.d. for 7 days, and carefully monitored for tolerance and pharmacokinetics. A preliminary assessment of efficacy can be made based on historical controls.
iii) A prospective, double blind, randomised phase II trial.

### 2. Physical, chemical and pharmaceutical properties

**Table 1:**

| | |
|---|---|
| Physical Form: | Aurograb is a white amorphous freeze-dried powder which, on solubilisation in water at 2 mg/mL, yields a clear, colourless solution. |
| Structural Formula: | Recombinant protein with the amino acid sequence of SEQ ID NO: 1 - a human scFv, together with a carboxy terminal histidine tag of 6 histidine amino acid residues. |
| Molecular Weight: | Monomer - 28.1 kDa - approximately 18 % of the molecule exists as a homodimer. |
| Characteristics: | A white powder or friable solid. Hygroscopic |
| Acidity/Alkalinity: | Aurograb is set at pH 9.5 ±0.2. |

### 3. Formulation, composition, handling & storage

### 3.1 Formulation & Composition

The complete composition of Aurograb is given in Table 2, below. It consists of lyophilised (freeze-dried) powder provided at 10 mg per vial. The drug is administered by resuspension of the powder in 5 mL sterile injectable water shortly prior to iv injection. No other solvent should be substituted for water as Aurograb is sensitive to pH fluctuations and has been especially formulated for resuspension in water.

**Table 2:**

| Names of ingredients | Quantity per Aurograb vial | Quantity per ml after reconstitution with 5 mL water | Function |
|---|---|---|---|
| Aurograb | 10 mg | 2 mg | Active Ingredient |
| Urea Ph Eur, BP, USP, JP | 150 mg | 30 mg | Excipient |
| Arginine Ph Eur | 174 mg | 34.8 mg | Excipient |

L-arginine and urea are present in order to maintain the pH balance of the formulation and ensure solubility of the Aurograb. Both are present in dosages which are considered safe for clinical intravenous administration. For example, a single dose of Aurograb (37.5 mL for 75 Kg patient body weight) contains 7.5 mmoles arginine. This is 26 times less than the maximum permitted daily dose of L-arginine when administered during the measurement of growth hormone levels in children (ABPI compendium, 1998-99). For urea, a single 35 mL dose of Aurograb contains 1.125 g urea. Again, this is much less than the 150 g urea maximum permitted daily dose of urea (this is the maximum dose indicated for the treatment of acute intracranial pressure *via* intravenous delivery - ABPI compendium, 1998-99).

### 3.2 Storage

The lyophilised drug is stored in glass vials at 4 °C in the dark. Overnight transport at ambient temperatures is acceptable. Avoid prolonged exposure to bright light or excessive humidity. No change in Aurograb activity after storage at 4 °C (3 months) has been observed.

After reconstitution with water, Aurograb must be stored at 4 °C and should be used within 14 hours. Activity will slowly deteriorate if left at room temperature.

### 3.3 Preparation and administration

Aurograb, provided at 10 mg per vial, is administered by resuspension of the powder in 5 mL of sterile injectable water shortly prior to intravenous (iv) injection. An example human dose is 1 mg/kg b.d. Sterile water is introduced directly into the vials *via* injection through the rubber cap with a hypodermic syringe/needle.

To reconstitute:
add *half* the final volume of water (2.5 ml per vial) and swirl to dissolve
filter (if necessary) to remove any undissolved material
add the remaining water.

NB. If administration delayed, store at 4 °C (for up to 14 hours).

No other solvent should be substituted for water as Aurograb is sensitive to pH fluctuations and has been especially formulated for resuspension in water.

Aurograb should be administered by slow iv bolus injection, for example through an iv giving set which should be flushed first with saline to remove traces of any other iv fluids and then again after administration of the drug.

No special precautions are required for the clean-up of spills or waste disposal.

Saline for iv use is compatible with reconstituted Aurograb (at a 50:50 dilution). It is less compatible with 20% glucose and sodium bicarbonate buffer, as shown by a reduction in ELISA activity, and therefore if being given via the same iv giving set as these or other fluids, the injection port must be flushed through with saline before and after administrating the Aurograb.

### 4. Non-clinical studies

Aurograb shows antibacterial activity, both *in vitro* and *in vivo,* against a wide range of *S. aureus* strains. In addition Aurograb has been shown to exert a synergistic antibacterial effect when used in conjunction with vancomycin.

### 4.1 Non-Clinical Efficacy Studies: in vitro data

Aurograb shows synergistic activity in combination with vancomycin against a wide range of MRSA strains, including strains of EMRSA with intermediate resistance to vancomycin ("VISA" - vancomycin insensitive MRSA)

These data were prepared using the same MIC methodology routinely applied to antibiotics.
- Aims:: To demonstrate synergy when used in combination with vancomycin, against a wide range of epidemic strains of MRSA.
- Objective:: Synergy is demonstrated by a reduction in the MIC of vancomycin when Aurograb is used in combination.

### Introduction

Epidemic strains of MRSA readily give rise to sub-population of intermediate resistance to vancomycin (i.e. MIC of 4 or 8 µg/ml) by serial passage in nutrient broth with increasing concentrations of vancomycin (Burnie J et al., Clin Infect Dis. 2000 Sep;31(3):684-9; PMID: 11017816). These are probably responsible for many of the treatment failures associated with vancomycin therapy. In this study Aurograb activity was examined against both these VISA and the fully vancomycin-sensitive parent EMRSA-15 strain.

### Method

Strains of MRSA were grown overnight in nutrient broth and diluted to give an inoculum of 1x10³ cells per mL. 100 µl aliquots of culture were placed into flat-bottomed microtitre plates and vancomycin added in the range 0.125 - 250 µg/ml. Cultures were incubated for a further 2 days. The minimum growth-inhibitory concentration (MIC) is defined as the highest concentration of antibiotic at which no bacterial growth occurs and is determined by spectrophotometric evaluation of the lack of turbidity (growth) in culture. MICs were determined in the presence of exogenous Aurograb or in the presence of Aurograb formulation buffer (negative control).

**Results: (Table 3)**

| MRSA Strain | MIC to Vancomycin in the presence of Aurograb at the following concentrations | | | |
|---|---|---|---|---|
| *VISA:-* | 0 µg/ml | 60 µg/ml | 80 µg/ml | 125 µg/ml |
| EMRSA-1 | 8 µg/ml | 4 µg/ml | 2 µg/ml | <0.125 µg/ml |
| EMRSA-2 | 4 µg/ml | 0.5 µg/ml | 1 µg/ml | <0.125 µg/ml |
| EMRSA-8 | 8 µg/ml | 2 µg/ml | 1 µg/ml | <0.125 µg/ml |
| EMRSA-11 | 4 µg/ml | 4 µg/ml | 1 µg/ml | <0.125 µg/ml |
| EMRSA-12 | 8 µg/ml | 4 µg/ml | 2 µg/ml | <0.125 µg/ml |
| EMRSA-15 | 8 µg/ml | 4 µg/ml | 2 µg/ml | <0.125 µg/ml |
| Parent EMRSA-15 | 1 µg/ml | - | 0.25 µg/ml | <0.125 µg/ml |

Spectrum of Activity: Enhanced antibacterial activity is evident in all strains tested demonstrating a broad spectrum of activity against strains of MRSA.

Synergy: Aurograb has the ability to increase the sensitivity of MRSA to vancomycin, including MRSA with reduced sensitivity to vancomycin.

**Further experiments** were undertaken to determine the MIC of vancomycin for a number of MRSA strains and the effect of the Aurograb antibody on MIC. The experiments were also performed using flucloxacillin instead of vancomycin to investigate how the efficacy of penicillin-type antibodies was affected by Aurograb. Results are given in Table 3a:

| EMRSA strain | MIC to vancomycin | MIC to vancomycin + 100 µg/ml Aurograb |
|---|---|---|
| 1 | 0.5 µg/ml | 0.03 µg/ml |
| 2 | 0.5 µg/ml | 0.03 µg/ml |
| 3 | 0.5 µg/ml | 0.03 µg/ml |
| 4 | 0.5 µg/ml | 0.03 µg/ml |
| 5 | 0.5 µg/ml | 0.03 µg/ml |
| 6 | 0.5 µg/ml | 0.0125 µg/ml |
| 7 | 0.5 µg/ml | 0.03 µg/ml |
| 8 | 0.5 µg/ml | 0.0125 µg/ml |
| 9 | 0.5 µg/ml | 0.0125 µg/ml |
| 10 | 0.5 µg/ml | 0.03 µg/ml |
| 11 | 0.5 µg/ml | 0.03 µg/ml |
| 12 | 0.5 µg/ml | 0.03 µg/ml |
| 13 | 0.5 µg/ml | 0.03 µg/ml |
| 14 | 0.25 µg/ml | 0.03 µg/ml |
| 15 | 0.5 µg/ml | 0.03 µg/ml |
| 16 | 0.5 µg/ml | 0.03 µg/ml |

Results obtained with flucloxacillin showed that the MIC obtained was > 256 µg/ml for all EMRSA strains. Flucloxacillin + Aurograb gave the same results and showed no decrease in the MIC of flucloxacillin.

**Conclusion**: Use of Aurograb in combination with vancomycin should both increase therapeutic efficacy and impede the emergence of vancomycin resistant strains. Due to the structure and function of the antibody with respect to the target molecule, similar results can be expected to be achieved using other glycopeptide antibiotics such as teicoplanin, the resistance mechanisms employed by *S. aureus* against them being the same as for vancomycin and therefore also being a target for the antibody and being susceptible to therapy using it.

### 4.2 Non-Clinical Efficacy Studies: in vivo data

The murine model of staphylococcal infection is widely used and is routinely used in the assessment of antimicrobial drugs. It is a good predictor of efficacy in infected humans since *S. aureus* is introduced intravenously to create a bacteraemia, from which the pathogen spreads to other organs, including the kidney, liver and spleen. Hence the nature of the infection (septicaemia) is analogous to the situation in infected patients. Also, the intravenous route of administration of Aurograb, used in the animal models (data given below) is the same route as that typically to be used in patients, improving the chances of comparable pharmacokinetics, drug bio-availability and efficacy.

### Data Set 1: Aurograb - intrinsic antibacterial activity against EMRSA-15

Aims: To demonstrate Aurograb is therapeutic when given alone in mice infected with a vancomycin-sensitive strain of MRSA, the EMRSA-15.

Objective: Anti-staphylococcal activity is demonstrated by a reduction in mortality or a reduction in bacterial load (colony forming units) in kidney, liver or spleen in the presence of Aurograb.

Method: 20 female CD-1 mice were given a sub-lethal challenge (1.3 x 10⁷ cfu) of a methicillin-resistant, vancomycin-sensitive strain of *S. aureus* (EMRSA-15, Typed Hospital Isolate, Central Manchester Healthcare Trust) in 100 µl iv bolus (all iv injections given via the lateral tail vein). 1 hour later, two groups of 10 mice, received, as 100 µl iv bolus:-
1. formulation buffer (Arginine-Urea), as negative control or
2. Aurograb (2.0 mg/kg) in formulation buffer.

All animals were sacrificed after a further 48 h and viable cfu per gram of organ were determined.

**Results: (Table 4)**

| Group (n=10) | Aurograb | Kidney | Liver | Spleen |
|---|---|---|---|---|
| | | Log cfu/g tissue | | |
| 1. Formulation Buffer | - | 8.58±2.59 | 4.79±0.53 | 5.04±0.24 |
| 2. Aurograb | 2 mg/kg | 7.20±1.15 | 3.63±0.68 | 3.79±0.82 |

### Conclusion:

Anti-bacterial activity - Viable staphylococci were found in the kidney, liver and spleen with preferential localisation in the kidney. Administration of Aurograb resulted in a log (10 fold) reduction in viable organisms found in all three organs. This suggests that Aurograb reduces the viability of *S. aureus* in experimental infections of mice in the absence of any other exogenous antimicrobial using the murine model of deep-seated infection.

### Data Set 2: Aurograb dose ranging study

Aims: Dose ranging study for use of Aurograb versus *S. aureus* strain EMRSA-15.

Objective: Antibacterial activity is demonstrated by the greater reduction in bacterial load (organ cfu) in kidney, liver or spleen when Aurograb is given, as a single agent, compared to the placebo control. Dose range is determined by comparing cfu (expressed as the log of the cfu per gram of tissue) for different organs at different doses.

Method: 40 female CD-1 mice (24-26 g) challenged with iv bolus of *S. aureus* followed 2 hours later by single iv dose of placebo or Aurograb (2 mg/kg, 1 mg/kg or 20 mg/kg). All mice terminated at 48 hours for culture of kidney, liver and spleen to determine organ viability counts.

### Results: expressed as log cfu per gram of tissue

Experiment 1: *S. aureus* (1.5 x 10⁷ cfu) : EMRSA-15 (Table 5)

| Aurograb dose | Kidney | Liver | Spleen |
|---|---|---|---|
| 0 | 8.40±2.256 | 7.22±2.52 | 6.98±2.39 |
| 2 mg/kg | 7.62±1.16 | 5.22±1.39 | 5.10±1.29 |
| 1 mg/kg | 8.7±2.6 | 5.40±1.38 | 5.33±1.21 |
| 0.2 mg/kg | 8.5±2.55 | 6.61±3.03 | 5.69±1.16 |

Experiment 2: *S. aureus* (9 x 10⁶ cfu) : clinical isolate EMRSA-15 - low dose Aurograb (Table 6)

| Aurograb dose | Kidney | Liver | Spleen |
|---|---|---|---|
| 0.2 mg/kg Aurograb | 7.14±1.28 | 3.13±0.44 | 3.40±0.64 |
| Placebo¹ | 7.36±1.05 | 4.14±1.27 | 4.01±1.26 |

| | | | |
|---|---|---|---|
| ¹ Negative control antibody (WC7) specific to a non-protective epitope of GrfA. | | | |

Experiment 1: a 10 to 100 fold reduction in viable bacteria for liver and spleen was achieved in the range 1.0 to 2.0 mg/kg Aurograb. At 0.2 mg/kg Aurograb, clearance from the spleen is still good, but reduced in the liver. The highest dose resulted in a 0.8 log reduction in kidney bacterial counts but no reduction occurred with the lower doses.

Experiment 2: At doses as low as 0.2 mg no reduction in viable staphylococci is evident in the kidneys, but there is evidence of bacterial killing in the liver and spleen.

Conclusion: Administration of Aurograb at 2 mg/kg results in a reduction in viable organisms found in all three organs, but particularly the spleen and liver. Aurograb at a dose of 1 mg/kg also showed significant antibacterial activity in the spleen and liver, but lost activity in the kidney. Doses as low as 0.2 mg/kg, still showed antibacterial activity in the spleen, and to a lesser extent, the liver.

Extrapolation to humans: A single dose of 2,0 mg/kg in mice gave an approximately 2.0 log drop in liver and spleen counts and a 0.8 log drop in kidney counts. This equates, on a body weight basis, to 1 mg/kg given twice daily to humans.

### Data Set 3: Aurograb antibacterial activity and synergy with vancomycin: sub-lethal model.

Aims: To demonstrate Aurograb is antibacterial when given alone and synergistic when given in conjunction with vancomycin in mice infected with a sublethal dose of *S. aureus.*

Objective: Antibacterial activity is demonstrated by a reduction in bacterial load (colony forming units) in kidney, liver or spleen in the presence of Aurograb alone. Synergy is demonstrated by a greater reduction in organ colony counts when Aurograb and vancomycin are given together than when either antibacterial is given alone.

Methods: 60 female CD-1 mice (22-24 g) were given 1 x 10⁷ cfu of EMRSA-15 as a 100 µl iv bolus. 2 hours later, in groups of 10 mice, they received, as a single 100 µl iv bolus:
Group 1 - 6.0 mg/kg vancomycin + 2.0 mg/kg Aurograb
Group 2 - 6.0 mg/kg vancomycin + 0.2 mg/kg Aurograb
Group 3 - 6.0 mg/kg vancomycin + placebo (formulation buffer)
Group 4 - placebo + 2.0 mg/kg Aurograb
Group 5 - placebo + 0.2 mg/kg Aurograb
Group 6 - placebo + 2.0 mg/kg Aurograb
All mice were terminated at 48 hours for culture of kidney, liver and spleen.

### Results: expressed as log cfu per gram of tissue, unless the majority of mice (>5) were cleared (ie organs were culture-negative). N/A = not applicable. (Table 7)

| Group | Vancomycin | Aurograb | Kidney | Liver | Spleen |
|---|---|---|---|---|---|
| | | | Log cfu/g of tissue (number of mice cleared) | | |
| 1 | 6 mg/kg | 2.0 mg/kg | 6.02±1.21 | 0 (all 10 clear) | N/A (9 clear) |
| 2 | 6 mg/kg | 0.2 mg/kg | 6.28±1.36 | N/A (7 clear) | 3.51±0.63 (5 clear) |
| 3 | 6 mg/kg | - | 6.53±1.53 | N/A (8 clear) | 3.63±0.91 (5 clear) |
| 4 | - | 2.0 mg/kg | 7.30±1.26 | N/A (8 clear) | 3.54±0.61(4 clear) |
| 5 | - | 0.2 mg/kg | 7.50±1.53 | 4.28±1.28 (2 clear) | 3.91±0.87 (3 clear) |
| 6 | - | - | 7.65±1.62 | 4.34±1.37 (1 clear) | 4.57±1.53 (1 clear) |

Alone: Aurograb administered alone at 2.0 mg/kg (group 4) gave comparable results to vancomycin alone (group 3), each clearing the livers of 8 mice and spleens of 4 and 5 mice respectively, compared to one mouse clearing the liver and spleen in the negative control group (group 6). Renal clearance was better with vancomycin than Aurograb, although none of the mice were cleared of renal infection.

Synergy Mice (group 1) receiving 2.0 mg/kg of Aurograb plus vancomycin (6 mg/kg) showed complete clearance of staphylococci from the livers of all 10 mice, and enhanced clearance from the spleens, being culture negative in 9 mice, compared to 4-5 being cleared by Aurograb or vancomycin alone and 1 being cleared in the untreated group. Renal colony counts in those receiving combination therapy (group 1) were reduced by an additional 0.5 log compared to mice receiving vancomycin alone (group 3), and reduced by 1.6 log compared to negative control mice (group 6). **This demonstrates the synergy between Aurograb and vancomycin.**

### Data Set 4: Aurograb synergy with vancomycin: lethality model.

Aims: To demonstrate Aurograb is antibacterial when given alone and synergistic when given in conjunction with vancomycin in mice infected with a lethal dose of *S. aureus.*

Objective: Antibacterial activity is demonstrated by a reduction in mortality with Aurograb alone. Synergy is demonstrated by a greater reduction in mortality when Aurograb is given in combination with vancomycin, rather than vancomycin alone.

Method: 60 female CD-1 mice (22-24 g) were given *S. aureus* (a fresh clinical isolate of EMRSA-15) at a dose of 8 x 10⁷ cfu in a 100 µl iv bolus. 2 hours later, in groups of 10 mice, they received, as a single 100 µl iv bolus:
Group 1 - 4.0 mg/kg vancomycin + 2.0 mg/kg Aurograb
Group 2 - 4.0 mg/kg vancomycin
Group 3 - 2.0 mg/kg vancomycin + 2.0 mg/kg Aurograb
Group 4 - 2.0 mg/kg vancomycin
Group 5 - 2.0 mg/kg Aurograb
Group 6 - placebo

All mice were terminated at 48 hours for culture of kidney, liver and spleen.
Mice fatalities prior to the 48 hour cull were not subject to organ colony counts.

### Results: expressed as log cfu per gram of tissue, unless the majority of mice (>5) were dead (Table 8)

| Group | Vancomycin | Aurograb | Survivors at 24 h (n=10) | Kidney | Liver | Spleen |
|---|---|---|---|---|---|---|
| | | | | Log cfu/g tissue | | |
| 1 | 4.0 mg/kg | 2.0 mg/kg | 8 | 7.25±1.32 | 4.55±1.43 | 4.24±0.96 |
| 2 | 4.0 mg/kg | - | 4 | Statistically too few survivors | | |
| 3 | 2.0 mg/kg | 2.0 mg/kg | 6 | 7.66±1.47 | 5.55±1.41 | 5.16±1.02 |
| 4 | 2.0 mg/kg | - | 5 | Statistically too few survivors | | |
| 5 | - | 2.0 mg/kg | 6 | 7.87±1 | 5.18±0.57 | 5.53±2.74 |
| 6 | - | - | 3 | Statistically too few survivors | | |

Mortality: This strain of *S. aureus,* probably because it was a fresh clinical isolate, was more virulent in the mouse model than the previously used laboratory strain of EMRSA-15, and a high degree of mortality was observed. Administration of Aurograb alone (2.0 mg/kg) was associated with a reduction in mortality (4 deaths) compared to the untreated group (7 deaths), comparable to the mortality on vancomycin alone (6 and 5 deaths). This suggests that Aurograb has comparable intrinsic anti-staphylococcal activity to vancomycin at 4.0 mg/kg.

Synergy: Mice receiving combination therapy (Aurograb 2.0 mg/kg and vancomycin 4.0 mg/kg) had higher rates of survival than with vancomycin or Aurograb alone suggesting a synergistic effect with the two drugs.

### 5. Pharmacokinetics and product metabolism in animals

### 5.1 Pharmacokinetics

The pharmacokinetic (PK) characteristics of Aurograb have been studied in mice. A murine model was used because efficacy and dose ranging studies have been performed in this species as were the repeat dose toxicology studies. Mice were given a single high dose as an iv bolus and then examined in duplicate for blood levels and organ distribution. Urine samples were also analysed. Samples were analysed for functional activity, measured by ELISA (blood samples only) and drug concentration (SDS PAGE/immunoblots).

### Results:

Results are shown in Figure 1. Mice given a single intravenous bolus injection of Aurograb (10 mg/kg) showed satisfactory blood levels.

All organs (lungs, brain, liver, spleen, heart, kidney and blood) were cleared by 24 hours.

### 6.2 Blood Compatibility Study

This study was carried out by Biochemical Pharmacology department at Inveresk Research, Scotland, in order to assess the suitability of Aurograb for intravenous administration.

Blood samples were taken from healthy human volunteers, transferred to tubes containing Aurograb, Aurograb vehicle, saponin or saline and incubated at 37 °C for 1 hour. After centrifugation, the amount of haemoglobin in the supernatant was then assessed to determine haemolysis. According to the American Society for Testing and Materials classification system (ASTM F756-93, 1993) the data indicated that 0.1 mg/ml Aurograb and the Aurograb vehicle were non-haemolytic. This compared with physiological saline which was non-haemolytic, and in total contrast to the positive reference compound (saponin) which was found to be severely haemolytic.

### 7 Manufacture of Aurograb antibody

### 7.1 Introduction

Aurograb is produced in *E.coli* in the form of inclusion bodies. The isolation, solubilisation, denaturation and refolding of inclusion bodies has been previously well documented (see 'Antibodies: A Laboratory Manual'. Harlow, E. and Lane, D. 1988. Cold Spring Harbor Laboratory Press; 'Inclusion bodies and purification of proteins in biologically active forms'. Mukhopadhyay, A.1997. Adv. Biochem. Eng. Biotechnol. ;56:61-109). An example of such a procedure is outlined in this application. Briefly, inclusion bodies were extracted from the cell mass, solubilised/denatured, refolded and purified using chromatography. Over expression of Aurograb is from the powerful T7 promoter in vector pET29b (Novagen). The cell mass is produced as a quality controlled, pure culture prepared in a sterile fermenter (1000L) using media free of animal products. The *E.coli* strain used is genetically disabled (unable to colonise/persist in the environment) and is avirulent. Impurities removed during downstream processing are thus (1) *E.coli* host cell proteins (HCPs) (2) *E.coli* pyrogens (mainly endotoxin) (3) fermentation additives (antibiotics) (4) downstream processing additives (buffers and NiSO₄).

Large amounts of contaminants from *E.coli* and virtually all fermentation media derived contaminants are removed during the process extraction of the protein from inclusion bodies. For this, the cell mass is isolated by centrifugation and broken using a micro fluidiser. The Aurograb inclusion bodies are separated from the cell mass by three centrifugation-resuspension- micro fluidisation steps thereby washing the inclusion bodies extensively. Inclusion bodies are solubilised and refolded over a two day period in the presence of copper chloride, which serves as an oxidation catalyst, thus establishing the correct and biologically active structure of the Aurograb molecule. Extensive diafiltration is performed to remove small molecules from the refolding reaction and trace residual kanamycin (from the fermentation reaction). Remaining host cell proteins are removed by immobilised nickel-metal affinity chromatography (IMAC) and anion exchange. In particular, endotoxin, which is a significant safety issue for drugs manufactured from *E.coli,* is efficiently removed during IMAC by the addition of deoxycholic acid. The last traces of *E.coli* HCP are removed during the anion exchange step. Traces of nickel from the IMAC step are removed using a nickel affinity column. Finally small molecules from the chromatography buffer system are removed using diafiltration against formulation buffer. The bulk product is lyophilised in vials for use in patients. The numbered paragraphs below specifically detail the manufacture of the Aurograb antibody.

### 7.2 Aurograb manufacturing protocol

Fermentation and downstream processing were performed using a total of nine steps as follows:
1. Fermentation
2. Harvest and isolation of inclusion bodies
3. Refolding
4. Concentration and diafiltration
5. Filtration and sterile filtration
6. Purification - Immobilised Nickel (Ni²⁺) metal affinity chromatography (IMAC)
7. Purification - Nickel removal by chelating sepharose chromatography
8. Purification - Anion exchange chromatography
9. Diafiltration and bulk filling

### 7.3 Fermentation

A frozen stock of the Aurograb expressing *E*. *coli* (Clone JM109 (DE3)(pSaABC4)) was inoculated into 4 x 500 ml glass conical flasks, to prepare the inoculum prior to transfer to a 1000L stirred fermenter (MBR). The precultures (supplemented with kanamycin) were incubated at 37 °C with shaking, for 15-20h, until the preculture OD₅₇₈= 4.0-8.0, representing 7-8 cell divisions. At production scale, the precultures were transferred into a 1000 L fermenter (MBH) containing 1000 L nutrient broth. The production culture was incubated at 37 °C for a total of 14-18h, representing 7 cell divisions. Approximately 10-13h post inoculation, when the OD₅₇₈= 5-8, the production culture was induced through the addition of 23.8g IPTG (100µM).

### 7.4 Harvest and isolation of inclusion bodies

Cells were harvested by centrifugation (4h post induction) at 12,800 g in an Alfa Laval BTPX 205 centrifuge with a flow rate of approx. 400 L/h. The harvested *E.coli* cell paste was stored at -50°C in PE bags (Kendro). The down stream processing of 100% cell paste mass from a single fermentation batch having been stored at -50°C constituted a single purification batch. The cell paste was thawed and resuspended in 1000L lysis buffer. The resuspended bacterial cells were subjected to high pressure homogenisation (APV Gaulin MC15) at ca. 1000 bar (15,000 psi) with a flowrate ca. 500 L/h. The inclusion bodies were sedimented by centrifugation at ca. 12.800 g with a flowrate ca. 400 L/h. The inclusion bodies were then washed with 400L of lysis buffer, and sedimented by centrifugation at ca. 12.800 g with a flowrate ca. 400 L/h, for a total of three times. The washed inclusion bodies were stored as a slurry at -70°C prior to the refolding step. The inclusion bodies were solubilised by strong agitation for 5-10 minutes in 40L 6M urea/100mM Tris pH 12.5 at room temperature.

### 7.5 Refolding

For refolding the inclusion body solution was made up to 560L with refolding buffer, and CuCl₂.2 H₂O was added to a final concentration of 100 µM. The preparation was incubated for 48h at 2-8°C under strong agitation. The inclusion bodies were filtered (Sartorius GF 30", 3.0/0.8 µm) and then filter sterilised (Seitz Supradisc SDPEK 1, depth filter).

### 7.6 Concentration and diafiltration

Concentration and diafiltration steps were carried out using cassettes (15m²) of omega screen channel with a 10kDa cutoff (Pall) housed in a "Centrasette" stainless steel holder (Pall), under the control of a 1000 membrane piston pump (pump quattro). The refolded protein preparation was concentration to 150L, followed by diafiltration with 3000 litres of 10 mM ammonium acetate buffer, pH 9.5. The preparation was adjusted to 6 M urea, 50 mM Tris, 10 mM DCA, 1 M NaCl and made up to a final volume of 300L with RO water. The diafiltered refold was stored over night at 2-8°C. The pH was then adjusted to 8.0 through the addition of HCl.

### 7.7 Filtration steps and sterile filtration

The diafiltered refold was filtered (Seitz Supradisc SDPEK1, depth filter) and then filter sterilised (Millipore Opticap 10", 0.2µm). The sterile product was stored at 2-8°C.

### 7.8 Immobilised Ni²⁺ metal affinity chromatography (IMAC)

The IMAC step was performed in a BPG300/500 column (Amersham Pharmacia). Sterilisation of chromatography columns took place during resin cleaning in place (CIP). IMAC chromatography resin (18L) was initally charged with 2 column volumes (CV) 100 mM NiSO₄.6H₂O. Chromatography utilised 5.5 (CV) equilibration buffer (IMAC A); 5.5 CV Wash buffer I (IMAC B); 3.3 CV Wash buffer II (IMAC C); 5.5 CV Elution buffer (IMAC D). Fractions collected from the eluate were filter sterilised (Millipore Opticap 10", 0.2µm) and stored overnight at 2-8°C.

### 7.9 Nickel removal chromatography

Nickel removal chromatography was performed in a BPG140/500 column (Amersham Pharmacia) using 2L chelating Sepharose resin. The column was equilibrated and washed with 5 CV buffer (IMAC D) and the Aurograb antibody containing solution was applied, the column was washed with 2CV anion/conditioning buffer (50 mM Tris, 6 M urea pH 8.7) and the eluate was retained prior to anion exchange chromatography.

### 7.10 Anion exchange chromatography

The Anion exchange chromatography step was performed in a BPG300/500 column (Amersham Pharmacia) using 18 L resin (Q-Sepharose FF). The column was equilibrated using equilibration buffer and then anion/conditioning buffer. The Aurograb antibody containing solution was applied, washed with 2CV anion/conditioning buffer, and the flow through was filter sterilised (Millipore Opticap 10", 0.2µm) and stored overnight at 2-8°C.

### 7.11 Diafiltration and bulk filling

The pH of the sterile flow through (containing the Aurograb antibody) was adjusted to 9.5, and was then diafiltered against 10 turnover volumes (TOV) 10 mM ammonium acetate; 0.5 M urea, pH 9.5. There followed another diafiltration step, against 5 TOV 0.5 M urea, 0.2 M arginine pH 9.5. The protein was concentrated to 2mg/ml, filter sterilised (Millipore Opticap 10", 0.2µm) into a Stedim bag, and stored at 2-8°C.

### 7.12 Composition of buffers, media and solutions

Growth media:
Media were monitored for microbial contamination prior to inoculation.
For 2.5 L preculture:

| | |
|---|---|
| Peptone A3 from soy bean (Organotechnie) | 67.5 g |
| Yeast autolysate (KAV; Deutsche Hefewerke) | 35.0 g |
| Sodium chloride (Ph.Eur, E.Merck) | 12.5 g |
| Glycerol (Ph.Eur, E.Merck) | 75.0 g |
| Dipotassium hydrogen phosphate (Ph.Eur, E.Merck) | 11.5 g |
| Potassium dihydrogen phosphate (Ph. Eur, E.Merck) | 7.5 g |
| Magnesium sulfate heptahydrate (Ph.Eur, E.Merck) | 1.25 g |
| Kanamycin sulfate (Sigma) | 62.5 mg |
| RO I water | 2.5 litres |
| pH | 7.1±0.1 |

The pre-weighed substances in the table above are dissolved in 2.5 L RO I-water. After sterilisation of the broth 10 ml kanamycin sulphate (62.5 mg in 50 ml WFI) was added.

| **Composition for 1000 L culture:** | |
|---|---|
| Peptone from soybean (Organotechnie) | 27.0 kg |
| Yeast autolysate (KAV; Deutsche Hefewerke) | 14.0 kg |
| Sodium chloride (Ph.Eur, E.Merck) | 5.0 kg |
| Glycerol (Ph.Eur, E.Merck) | 30.0 kg |
| Disodium hydrogen phosphate (Ph.Eur, E.Merck) | 4.6 kg |
| Sodium dihydrogen phosphate (Ph. Eur, E.Merck) | 3.0 kg |
| Magnesium sulphate heptahydrate (Ph.Eur, E.Merck) | 0.5 kg |
| Struktol (antifoam agent, Sichler) | 600 ml |
| Kanamycin sulfate (USP, Sigma) | 25.0 g |
| RO I water | 1000 litres |
| pH | 7.0±0.2 |

The 1000 L fermenter was sterilised for 30 minutes at 121°C.

**IPTG:**
23.8 g IPTG (Sigma) was dissolved in 200 ml WFI and sterile filtered (0.2 µm).

| **Cell lysis buffer** | |
|---|---|
| Tris (Ph.Eur, E.Merck) | 6.06 gL⁻¹ |
| EDTA (Ph.Eur, E.Merck) | 0.37 gL⁻¹ |
| Potassium chloride (DAB, E.Merck) | 7.46 gL⁻¹ |
| RO I water | 1000 litres |
| pH | 8.0±0.1 |

| **Refolding buffer** | |
|---|---|
| Tris (DAB, E.Merck) | 5.96 gL⁻¹ |
| Copper II chloride (p.a., E.Merck) | 0.018 gL⁻¹ |
| RO I water (1000 L stainless steel container) | 600 litres |
| pH | 9.0±0.1 |

| **Diafiltration buffer:** | |
|---|---|
| Ammonium acetate (E.Merck) | 0.77 gL⁻¹ |
| RO I water | 1000 litres |
| pH | 9.0±0.1 |

| **IMAC buffers:** | |
|---|---|
| Sample Preparation | |
| Tris | 1.82 kg |
| urea | 108 kg |
| sodium chloride | 17.5 kg |
| deoxycholic acid, sodium salt | 1.30 kg |

The composition of the 150 L diafiltered refolding reaction was adjusted with the above reagents.

**IMAC chromatography buffers:**
Nickel solution: 100 mM NiSO₄.6 H₂O (2CV)
IMAC A/Equilibration buffer: 100 mM Tris; 6 M urea; 1M NaCl; 10 mM DCA, pH 8.0 IMAC B/Wash buffer: 100 mM Tris; 6 M urea; 1M NaCl; 50 mM imidazole; 10 mM DCA, pH 8.0
IMAC C/Elution buffer I: 100 mM Tris; 6 M urea; 1M NaCl; 150 mM imidazole; pH 8.0 (10-15 CV)
IMAC D/Elution II: 100 mM Tris; 6 M urea; 1M NaCl; 300 mM imidazole pH 8.0 (7 CV) CIP: 1 M NaOH (3 column volumes, incubation time 1 hour)
Buffers were prepared with Tris (USP, E.Merck), Urea (USP, E.Merck or Riedel de Haen), NaCl (Ph.Eur, E.Merck), Imidazole (p.a., Fluka), DCA (Deoxycholic acid, sodium salt monohydrate, Microselect, Fluka), NiSO₄.6 H₂O (p.a., E.Merck)

**Ni²⁺ Removal Chromatography buffers:**
Equilibration buffer: 100 mM Tris; 6 M urea; 50 mM NaCl; 300 mM imidazole; pH 8.0 (5CV)

**Anion exchange chromatography buffers:**
Equilibration: 100 mM Tris; 6 M urea; 50 mM NaCl; 300 mM Imidazole pH 8.7
CIP: 1 M NaOH (3CV, incubation time 1 hour)

### Diafiltration and Formulation Buffer

For sterilisation and storage, the diafiltration cassettes were flushed with RO water, recirculated for at least 45 minutes with 0.5 M NaOH and stored with 0.1 M NaOH.
Diafiltration buffer: 0.5 M urea; 10 mM ammonium acetate pH 9.5
Diafiltration (formulation) buffer: 0.5 M urea; 0.2 M arginine pH 9.5
Diafiltration and concentration (2 cassettes, Omega screen channel, 10 KD, Pall)

### 8. Determination of Synergistic Effect of Aurograb with Vancomycin

### 8.1 Method

In order to determine the Minimum Inhibitory Concentration (MIC), the Fractional Inhibitory Concentration (FIC) and the FIX (fractional inhibitory index), the following was method was used.

Representatives of each of the most common EMRSA strains, a number of vancomycin resistant subpopulations, and three Linzolid resistant strains were grown overnight in nutrient broth. The cells were diluted in Isosensitest Broth (Oxoid, UK) to give a final inoculum of 1x10³ cells per mL. 100 µl aliquots of culture were placed into flat-bottomed microtitre plates and vancomycin added in the range 0.125 - 250 µg/ml. To this was added exogenous Aurograb in the range 0.3 - 25 µg/ml in a checkerboard formation. A negative control series was also prepared containing Aurograb formulation buffer alone. Cultures were incubated for 48 hours at 37 °C.

The minimum inhibitory concentration (MIC) was defined as the lowest concentration of agent at which no bacterial growth occurred and was determined by spectrophotometric evaluation of the lack of turbidity (growth) in culture. This optical clearing was equivalent to >99% Staphylococcal death.

### 8.2 Results

MIC values were obtained for vancomycin and Aurograb (RTM) alone and in combination. Fractional inhibitory concentrations (FIC) and FIX values were calculated. Synergistic activity between the two agents was defined as an FIX value ≤0.5. Indifferent activity was defined as a value of 0.5 - 4, and a value of ≥4 defined antagonism.

Results of the experiment are given below in Tables 9 and 10. VAN indicates vancomycin. The MIC (Alone) column indicates the minimum inhibitory concentration of the agent when administered alone. The MIC (In combination) column indicates the minimum inhibitory concentration of each drug when used in combination.

### 8.3 Conclusions

As can be seen from Tables 9 and 10, the administration of Aurograb with vancomycin results in a synergistic therapeutic effect being achieved, with MIC levels being substantially reduced, which in turn indicates that therapy of patients can be effected with lower dosages of antibiotics and to greater therapeutic effect, with an expected reduction in possible side-effects resulting from the antibiotic. The results indicae that even in cases where a patient is infected with a vancomycin-resistant *S. aureus* they may be successfully treated with the antibody of the present invention and its use in combination with antibiotics such as vancomycin may enable effective therapy at previously ineffectual nontoxic dosage levels of antibiotic.

**Table 9:**

| EMRSA Strain | Agent | MIC (µg/ml) of each agent) | | FIC (µg/ml) | FIX | Outcome |
|---|---|---|---|---|---|---|
| | | Alone | In combination | | | |
| 1 | VAN | 0.5 | 0.03 | 0.06 | 0.066 | Synergy |
| | Aurograb | 125 | 0.75 | 0.006 | | |
| 1A | VAN | 8 | 1 | 0.125 | 0.17 | Synergy |
| | Aurograb | 250 | 12.5 | 0.05 | | |
| 2 | VAN | 0.5 | 0.03 | 0.06 | 0.07 | Synergy |
| | Aurograb | 125 | 0.75 | 0.006 | | |
| 2A | VAN | 8 | 1 | 0.125 | 0.17 | Synergy |
| | Aurograb | 250 | 12.5 | 0.05 | | |
| 3 | VAN | 0.5 | 0.03 | 0.06 | 0.07 | Synergy |
| | Aurograb | 250 | 3 | 0.012 | | |
| 4 | VAN | 0.5 | 0.03 | 0.06 | 0.06 | Synergy |
| | Aurograb | 125 | 0.325 | 0.0026 | | |
| 5 | VAN | 0.5 | 0.03 | 0.06 | 0.06 | Synergy |
| | Aurograb | 250 | 0.325 | 0.001 | | |
| 6 | VAN | 0.5 | 0.125 | 0.025 | 0.03 | Synergy |
| | Aurograb | 125 | 0.325 | 0.0026 | | |
| 7 | VAN | 0.5 | 0.03 | 0.06 | 0.06 | Synergy |
| | Aurograb | 125 | 0.325 | 0.0026 | | |
| 8 | VAN | 0.5 | 0.03 | 0.025 | 0.03 | Synergy |
| | Aurograb | 250 | 0.325 | 0.0013 | | |
| 8A | VAN | 0.5 | 0.03 | 0.06 | 0.11 | Synergy |
| | Aurograb | 250 | 12.5 | 0.05 | | |
| 9 | VAN | 0.5 | 0.0125 | 0.025 | 0.03 | Synergy |
| | Aurograb | 125 | 0.75 | 0.006 | | |
| 10 | VAN | 0.5 | 0.03 | 0.06 | 0.07 | Synergy |
| | Aurograb | 125 | 1.5 | 0.012 | | |
| 11 | VAN | 0.5 | 0.03 | 0.06 | 0.07 | Synergy |
| | Aurograb | 125 | 0.75 | 0.006 | | |
| 11A | VAN | 4 | 1 | 0.25 | 0.30 | Synergy |
| | Aurograb | 250 | 12.5 | 0.05 | | |
| 12 | VAN | 0.5 | 0.03 | 0.06 | 0.07 | Synergy |
| | Aurograb | 250 | 0.325 | 0.0013 | | |
| 12A | VAN | 0.5 | 0.03 | 0.06 | 0.06 | Synergy |
| | Aurograb | 250 | 0.75 | 0.003 | | |
| 13 | VAN | 0.5 | 0.03 | 0.06 | 0.07 | Synergy |
| | Aurograb | 250 | 1.5 | 0.006 | | |
| 14 | VAN | 0.5 | 0.03 | 0.06 | 0.08 | Synergy |
| | Aurograb | 125 | 1.5 | 0.012 | | |
| 15 | VAN | 0.5 | 0.03 | 0.06 | 0.07 | Synergy |
| | Aurograb | 125 | 1.5 | 0.012 | | |
| 15A | VAN | 0.5 | 0.03 | 0.06 | 0.07 | Synergy |
| | Aurograb | 250 | 0.75 | 0.006 | | |
| 16 | VAN | 0.5 | 0.03 | 0.06 | 0.07 | Synergy |
| | Aurograb | 250 | 1.5 | 0.012 | | |
| 17 | VAN | 4 | 1 | 0.25 | 0.25 | Synergy |
| | Aurograb | 250 | 0.75 | 0.006 | | |

**Table 10:**

| EMRSA Strain | Agent | MIC (µg/ml) of each agent) | | FIC (µg/ml) | FIX | Outcome |
|---|---|---|---|---|---|---|
| | | Alone | In combination | | | |
| NARSA 1 (Hiramatsu) | VAN | 8 | 2 | 0.25 | 0.25 | Synergy |
| | Aurograb | 250 | 0.325 | 0.001 | | |
| NARSA 2 (Hiramatsu) | VAN | 3 | 0.5 | 0.17 | 0.17 | Synergy |
| | Aurograb | 250 | 0.325 | 0.001 | | |
| NARSA 12 (France) | VAN | 8 | 1 | 0.12 | 0.12 | Synergy |
| | Aurograb | 250 | 0.75 | 0.003 | | |
| NARSA 17 (New York) | VAN | 8 | 2 | 0.25 | 0.25 | Synergy |
| | Aurograb | 250 | 0.325 | 0.001 | | |
| NARSA 119 (Linezolid R) | VAN | 4 | 1 | 0.25 | 0.27 | Synergy |
| | Aurograb | 125 | 3 | 0.02 | | |
| NARSA 120 (Linezolid R) | VAN | 4 | 1 | 0.25 | 0.27 | Synergy |
| | Aurograb | 125 | 3 | 0.02 | | |
| NARSA 121 (Linezolid R) | VAN | 4 | 1 | 0.25 | 0.27 | Synergy |
| | Aurograb | 125 | 3 | 0.02 | | |

The invention will now be described by way of a series numbered of clauses. CLAUSES
1. An antibody having the sequence of SEQ ID NO: 2 or an antigen binding fragment thereof.
2. An antibody specific for the epitope displayed by the peptide having the amino acid sequence of SEQ ID NO: 3 for use in a method of treatment or diagnosis of the human or animal body.
3. The use of an an antibody specific for the epitope displayed by the peptide having the amino acid sequence of SEQ ID NO: 3 in a method of manufacture of a medicament for the treatment of infection.
4. A medicament, comprising a therapeutically effective quantity of a glycopeptide antibiotic and antibody specific against GrfA.
5. A medicament according to clause 4, for use in the treatment or diagnosis of the human or animal body.
6. A medicament according to clause 4 being for the treatment of infection by gram positive bacteria.
7. A method of manufacture of a medicament for the treatment of infection, characterised in the use of a therapeutically effective quantity of a glycopeptide antibiotic and antibody specific against GrfA.
8. A pharmaceutical pack for the treatment of infection, comprising a therapeutically effective quantity of a glycopeptide antibiotic and antibody specific against GrfA.
9. A method of treatment of infection, comprising the step of administering to a patient in need of same a therapeutically effective quantity of a glycopeptide antibiotic and antibody specific against GrfA.
10. A medicament, method of manufacture, pharmaceutical pack or method of treatment according to any one of clauses 4 and 7-9, said antibody having the sequence of SEQ ID NO: 2
11. A medicament, method of manufacture, pharmaceutical pack or method of treatment according to any one of clauses 4 and 7-10, said antibody being specific against the epitope of SEQ ID NO: 3.
12. A medicament, method of manufacture, pharmaceutical pack or method of treatment according to any one of clauses 4 and 7-11, said glycopeptide antibiotic being selected from the group consisting of vancomycin, teicoplanin and daptomycin.
13. A medicament, method of manufacture, pharmaceutical pack or method of treatment according to any one of clauses 4 and 7-12, said infection being of a gram positive bacterium.
14. A medicament, method of manufacture, pharmaceutical pack or method of treatment according to clause 13, said bacterium being a Staphylococcus.
15. A medicament, method of manufacture, pharmaceutical pack or method of treatment according to clause 14, said bacterium being a coagulase-negative Staphylococcus.
16. A medicament, method of manufacture, pharmaceutical pack or method of treatment according to clause 15, said Staphylococcus being selected from the group consisting of *S. haemolyticus*, *S. epidermidis* and *S. saprophyticus.*
17. A medicament, method of manufacture, pharmaceutical pack or method of treatment according to clause 14, said bacterium being a coagulase-positive Staphylococcus.
18. A medicament, method of manufacture, pharmaceutical pack or method of treatment according to clause 17, said Staphylococcus being *S*. *aureus.*
19. A medicament, method of manufacture, pharmaceutical pack or method of treatment according to clause 13, said bacterium being selected from the group consisting of *Enterococcus sp., Enterococcus faecalis, Enterococcus faecium, Corynebacterium sp., Corynebacterium jeikeium,* and *Corynebacterium xerosis.*
20. A medicament, method of manufacture, pharmaceutical pack or method of treatment according to clause 13, said bacterium being resistant to treatment by said glycopeptide antibiotic alone.

## Claims

1. A medicament, comprising a therapeutically effective quantity of daptomycin and antibody specific against the epitope displayed by the peptide having the amino acid sequence of SEQ. ID NO: 3.

2. A medicament according to claim 1 for use in the treatment or diagnosis of the human or animal body.

3. A medicament according to claim 1, for use in the treatment of infection by gram-positive bacteria.

4. A method of manufacture of a medicament for the treatment of infection, **characterised in** the use of a therapeutically effective quantity of daptomycin and antibody specific against the epitope displayed by the peptide having the amino acid sequence of SEQ. ID NO: 3.

5. A pharmaceutical pack for the treatment of infection, comprising a therapeutically effective quantity of daptomycin and antibody specific against the epitope displayed by the peptide having the amino acid sequence of SEQ. ID NO: 3.

6. A medicament, method of manufacture, or pharmaceutical pack according to any one of the preceding claims, wherein said antibody has the sequence of SEQ. ID NO: 2 or an antigen binding fragment thereof.

7. A method of manufacture, or pharmaceutical pack according to any one of claims 4 to 6, said infection being of a gram-positive bacterium.

8. A medicament method of manufacture, or pharmaceutical pack according to claim 3 or 7, said bacterium being a Staphylococcus.

9. A medicament, method of manufacture, or pharmaceutical pack according to claim 8, said bacterium being a coagulase-negative Staphylococcus.

10. A medicament, method of manufacture, or pharmaceutical pack according to claim 9, said Staphylococcus being selected from the group consisting of *S. haemolyticus, S. epidermidis* and *S. saprophyticus.*

11. A medicament, method of manufacture, or pharmaceutical pack according to claim 8, said bacterium being a coagulase-positive Staphylococcus.

12. A medicament, method of manufacture, or pharmaceutical pack according to claim 11, said Staphylococcus being *S.aureus.*

13. A medicament, method of manufacture, or pharmaceutical pack according to claim 3 or 7 said bacterium being selected from the group consisting of *Enterococcus sp., Enterococcus faecalis, Enterococcus faecium, Corynebacterium sp., Corynebacterium jeikeium,* and *Corynebacterium xerosis.*

14. A medicament, method of manufacture, or pharmaceutical pack according to claim 3 or 7, said bacterium being resistant to treatment by daptomycin alone.

15. An antibody having the sequence of SEQ. ID NO: 2 or an antigen binding fragment thereof.
